# EUROPÄISCHE PATENTSCHRIFT

(11) **EP 3 630 473 B1**
(45) Veröffentlichungstag und Bekanntmachung des Hinweises auf die Patenterteilung: **21.08.2024**
(21) Anmeldenummer: 18725825.6
(22) Anmeldetag: 17.05.2018
(51) Int. Cl.: B29D 11/02, A61F 2/16

(54) **KÜNSTLICHE AUGENLINSE MIT DARIN AUSGEBILDETEM MEDIKAMENTENDEPOT UND VERFAHREN ZUM HERSTELLEN EINER KÜNSTLICHEN AUGENLINSE**
ARTIFICIAL EYE LENS HAVING MEDICINE REPOSITORY FORMED THEREIN, AND METHOD FOR PRODUCING AN ARTIFICIAL EYE LENS
CRISTALLIN ARTIFICIEL RENFERMANT UN MÉDICAMENT POUR UNE LIBÉRATION PROLONGÉE ET PROCÉDÉ DE FABRICATION D'UN CRISTALLIN ARTIFICIEL

(30) Priorität: 01.06.2017 DE 102017112085
(43) Veröffentlichungstag der Anmeldung: 08.04.2020
(73) Patentinhaber: Carl Zeiss Meditec AG, 07745 Jena (DE)
(72) Erfinder: KASCHKE, Michael, 73447 Oberkochen (DE); DICK, Manfred, 07926 Gefell (DE); GERLACH, Mario, 16548 Glienicke-Nordbahn (DE)
(74) Vertreter: Hofstetter, Schurack & Partner
(86) Internationale Anmeldenummer: PCT/EP2018/062921
(87) Internationale Veröffentlichungsnummer: WO 2018/219670

(56) Entgegenhaltungen:
- US-A1- 2006 235 514
- US-A1- 2006 246 112
- US-A1- 2015 073 549
- US-A1- 2015 342 725
- US-A1- 2015 378 065
- US-A1- 2016 331 519
- US-A1- 2017 119 521

## Beschreibung

### Technisches Gebiet

Die Erfindung betrifft eine künstliche Augenlinse mit einem optischen Teil, der eine in Richtung einer optischen Hauptachse der Augenlinse betrachtet erste optische Seite und eine gegenüberliegende zweite optische Seite aufweist. Die multifokale, künstliche Augenlinse weist darüber hinaus eine Haptik auf, mittels welcher die Augenlinse in einem Auge positionierbar ist. Die Augenlinse weist eine den optischen Teil zumindest teilweise umgebende und zur Haptik unterschiedliche Einfassung auf. In der Haptik und/oder dieser Einfassung ist eine Struktur mit zumindest einer Vertiefung ausgebildet. Des Weiteren betrifft die Erfindung auch ein Verfahren zum Herstellen einer derartigen Linse mittels eines Lasers.

### Stand der Technik

Multifokale, künstliche Augenlinsen sind aus dem Stand der Technik vielfältigst bekannt. Insbesondere sind dazu Intraokularlinsen bekannt, die die natürliche Linse im Auge ersetzen und diesbezüglich implantiert sind.

Aus der US 2006/246112 A1 ist eine Intraokularlinse mit Perforationen im Haptikplatten bekannt.

Aus der US 2010/0082017 A1 ist eine Intraokularlinse bekannt, bei welcher in einem haptischen Teil als auch in einem optischen Teil Schlitze ausgebildet sind, um die mechanische Charakteristik und auch die Strukturcharakteristik der Linse zu modifizieren. Diese länglichen Schlitze sind im Inneren der Intraokularlinse ausgebildet, insbesondere mit einem Laser.

Darüber hinaus ist aus der US 2004/0032566 A1 ein Verfahren zur Markierung einer Intraokularlinse mittels Laser bekannt. Mit dem Laser wird eine Mikroperforierung des optischen Teils der Linse durchgeführt.

Bekannt ist es auch, dass bei der Implantation von künstlichen Augenlinsen Komplikationen auftreten können, die bei der Operation, während des Ausheilprozesses nach der Operation oder auch gegebenenfalls erst später auftreten können. Dazu ist dann gegebenenfalls zusätzlich wiederum eine Nachbehandlung mit einem chirurgischen Eingriff erforderlich, um die Beeinträchtigungen ausheilen zu können.

### Darstellung der Erfindung

Es ist eine Aufgabe der Erfindung, eine künstliche Augenlinse zu schaffen, deren Funktionalität verbessert ist. Des Weiteren ist es Aufgabe der Erfindung, ein Verfahren zu schaffen, um eine derartige Linse herstellen zu können.

Ein Aspekt der Erfindung betrifft eine künstliche Augenlinse, die einen optischen Teil aufweist, mittels welchem die optische Abbildungseigenschaft der Augenlinse charakterisiert ist. Dieser optische Teil weist eine erste optische Seite und eine in Richtung der optischen Hauptachse dieser Augenlinse betrachtet gegenüberliegende zweite optische Seite auf. Die künstliche Augenlinse weist des Weiteren eine Haptik auf. Mit der Haptik ist die Augenlinse positionell in einem Auge gehalten. Die künstliche Augenlinse weist zusätzlich zur Haptik eine den optischen Teil zumindest bereichsweise umgebende und zur Haptik unterschiedliche Einfassung auf. Diese Einfassung ist dann weder Bestandteil des optischen Teils noch ist sie Bestandteil insbesondere von Bügeln einer Haptik, wenn eine derartige Haptik vorhanden ist. Die künstliche Augenlinse weist eine Struktur auf, die zumindest eine Vertiefung aufweist. Die Struktur ist als Mikroperforation mit einer Vielzahl von einzelnen und separaten

Perforationszonen ausgebildet. Als Mikroperforation ist eine Perforation zu verstehen, die Perforationen mit einer Längserstreckung im Bereich von Mikrometern und/oder im Bereich von Nanometern aufweist. Zumindest einige Perforationszonen sind als Medikamentendepot ausgebildet und sind mit zumindest einem Medikament zumindest bereichsweise gefüllt.

Durch eine derartige Ausgestaltung ist eine künstliche Augenlinse geschaffen, die in sich selbst zumindest ein Medikament beinhaltet. Die Funktionalität der Augenlinse ist dadurch erhöht. Neben ihrer primären Aufgabe, das optische Sehvermögen eines Auges zu verbessern, ist durch diese Ausführungsform der Erfindung auch noch die zusätzliche Funktion inhärent, als Medikamententräger zu dienen. Durch eine derartige künstliche Augenlinse ist es somit auch erreicht, dass für eine medikamentöse Behandlung während eines operativen Eingriffs insbesondere zum Implantieren einer als Intraokularlinse ausgebildeten künstlichen Augenlinse, und/oder beim Heilungsprozess nach einem derartigen operativen Eingriff und/oder für eine dann auch nach dem Ausheilungsprozess nachhaltige Aufrechterhaltung des Gesundheitszustands das Medikament im Auge bereits jeweils vorhanden ist. Das Medikament kann dann auch individuell ausgegeben werden, indem es aus einer Perforationszone austritt. Dies kann durch Wechselwirkung beispielsweise mit einem oder mehreren Medien im Auge, insbesondere im Kapselsack, ohne Einwirkung von außen hervorgerufen werden. Allgemein kann diese Abgabe eines Medikaments aus einer Perforationszone abhängig von den Umgebungsbedingungen im Auge erfolgen. In dem Zusammenhang kann auch ein Verschluss einer Perforationszone, der insbesondere nach dem Befüllen der Perforationszone mit zumindest einem Medikament, erzeugt ist, entfernt werden, in dem er sich beispielsweise auflöst. Beispielsweise kann abhängig von der Dicke des Verschlusses und/oder dem Werkstoff dieses Entfernen im implantierten Zustand in einem Auge auch bezüglich des Zeitpunktes und/oder der Zeitdauer bereits bei der Herstellung der künstlichen Augenlinse definiert eingestellt werden. Dies bedeutet auch, dass der Zeitpunkt der Medikamentenabgabe und/oder die Zeitdauer der Medikamentenabgabe aus einer Perforationszone im implantierten Zustand der künstlichen Augenlinse individuell eingestellt werden kann. Zusätzliche operative Eingriffe insbesondere nach dem Implantieren einer derartigen Augenlinse in das Auge, um einen Heilungsprozess zu begünstigen oder den ausgeheilten Zustand nachhaltig aufrecht zu erhalten, sind daher nicht mehr erforderlich oder nur noch in eingeschränktem Maße beziehungsweise in Ausnahmefällen erforderlich. Die Beeinträchtigung eines Patienten mit Folgeeingriffen kann daher deutlich reduziert werden.

Insbesondere ist eine derartige Struktur und somit auch eine Perforationszone als Laserstruktur ausgebildet. Dies bedeutet, dass sie mit einem Laser erzeugt ist. Dadurch kann die Perforationszone äußerst ortsgenau und/oder äußerst geometriegenau erzeugt werden. Unerwünschte Maßtoleranzen und/oder unerwünschte Beeinträchtigungen spezifischer Bereiche der künstlichen Augenlinse, insbesondere des optischen Teils, sind dadurch verhindert.

Durch dieses Ausbilden der Mikroperforation in zum optischen Teil unterschiedlichen Bereich der künstlichen Augenlinse wird der optische Teil als solcher auch nicht beeinträchtigt. Die optische Abbildungseigenschaft bleibt daher unbeeinträchtigt.

Mit zumindest einem derartigen Medikamentendepot ist es dann auch ermöglicht, dass das zeitliche Ausgeben des zumindest einen Medikaments aus dem Medikamentendepot individuell erfolgen kann, sodass situationsabhängig für den jeweiligen Heilungsvorgang gezielt auch sehr schnell eine bedarfsgerechte gegebenenfalls sehr große Menge eines Medikaments aus einem Medikamentendepot im implantierten Zustand der Augenlinse ausgegeben werden kann. Ebenso ist es möglich, dass dieses Ausgeben des Medikaments aus einem Medikamentendepot nach der Implantation der Augenlinse in das Auge über einen längeren Zeitraum und auch kontinuierlich erfolgen kann.

In einer vorteilhaften Ausführung kann vorgesehen sein, dass ein erstes Medikament in zumindest einer ersten Perforationszone, die dann ein Medikamentendepot darstellt, eingebracht ist und ein dazu unterschiedliches zweites Medikament in zumindest einer dazu unterschiedlichen zweiten Perforationszone, die dann auch wiederum ein eigenes Medikamentendepot darstellt, eingebracht ist. Es kann auch vorgesehen sein, dass zumindest zwei unterschiedliche Medikamente in einem gemeinsamen Medikamentendepot eingebracht sind. Ebenso ist es möglich, dass mehr als zwei unterschiedliche Medikamente in einer Perforationszone als Medikamentendepot eingebracht sind oder zumindest drei Medikamente jeweils in unterschiedlichen eigenen separaten Perforationszonen untergebracht sind. Ebenso ist es möglich, dass in einer Perforationszone nur ein spezifisches Medikament eingebracht ist und in zumindest einer weiteren, dazu unterschiedlichen Perforationszone zumindest zwei voneinander verschiedene Medikamente eingebracht sind. Abhängig davon, welche für den individuellen Patienten gegebenenfalls bevorzugte Medikamentenbehandlung und/oder bevorzugt über welchen Zeitraum eine derartige Medikamentenbehandlung erforderlich ist oder erforderlich sein wird, kann dann auch eine individuelle medikamentös künstliche Gestaltung dieser Medikamentendepots bereits vor dem operativen Eingriff zum Einbringen der Augenlinse in das Auge vorgenommen werden. So kann in dem Zusammenhang beispielsweise auf unterschiedliche Vorerkrankungen eines Auges und/oder eines Patienten eingegangen werden, beispielsweise wenn ein Patient mit Diabetes und/oder Bluthochdruck etc. mehr oder minder stark erkrankt ist. Es kann somit auch ermöglicht werden, dass eine derartige künstliche Augenlinse sehr patientenindividuell gestaltet wird und abhängig davon, wie der Patient gesundheitlich in seiner Konstitution ausgebildet ist, sehr individuell und bedarfsgerecht dann die Gestaltung dieser Medikamentendepots erfolgen.

Vorzugsweise ist vorgesehen, dass die mit zumindest einem Medikament zumindest bereichsweise gefüllten Perforationszonen als Sacklöcher ausgebildet sind. Dadurch lässt sich einerseits eine sehr individuelle und präzise Volumenvorgabe, insbesondere durch einen Laser, erzeugen, sodass auch die Medikamentenmenge, die in eine derartige Perforationszone eingebracht wird, sehr genau erfolgen kann. Darüber hinaus ist durch eine solche Ausgestaltung das unerwünschte Austreten eines Medikaments in eine nicht vorgesehene Richtung verhindert. Das Freigeben beziehungsweise Austreten des Medikaments aus der Perforationszone kann in dieser Form dann nur in die Richtung erfolgen, in welcher das Sackloch seine Sacklochöffnung aufweist.

Bevorzugt sind die Sacklöcher im Querschnitt trichterförmig ausgebildet, sodass mit zunehmender Tiefe die Querschnittsfläche eines Sackloches abnimmt. Am oberen Rand des Sackloches besteht somit eine geringere Kapillarität als am Boden des Sackloches. Dies ist vorteilhaft, da somit in Abhängigkeit von dem Öffnungswinkel des trichterförmigen Sackloches eine unterschiedlich schnelle Abgabe eines Medikamentes aus dem Sackloch erreichbar ist.

Es kann vorgesehen sein, dass die Mikroperforationen und somit die Perforationszonen bezüglich ihrer Orientierung gleichgerichtet sind oder zumindest zwei verschiedene Perforationszonen auch mit ihren Längsachsen der Perforationszonen unterschiedlich orientiert sind. Auch dadurch kann abhängig davon, welche Medikamente und/oder an welchen Stellen die Medikamente, insbesondere hauptsächlich, ihre Wirkung entfalten sollen, eine diesbezüglich individuell gestaltete Struktur geschaffen werden. Auch dadurch lässt sich erreichen, dass allein schon die Strukturierung gegebenenfalls dem jeweiligen individuellen Anforderungsprofil angepasst erzeugt ist, insbesondere patientenangepasst ausgebildet ist. Dies bedeutet auch, dass die Anzahl und/oder die Lage und oder die Orientierung der Perforationszonen und/oder deren Positionen individuell erzeugt werden können.

Auch kann dies dann derart erfolgen, dass die Perforationszonen örtlich spezifiziert an der Haptik und/oder der Einfassung ausgebildet sind, wobei dies abhängig davon erfolgt, welche Medikamente und/oder welche Menge der Medikamente für diese individuelle Ausgestaltung der Augenlinse erforderlich sind.

Die künstliche Augenlinse ist vorzugsweise multifokal, also zumindest bifokal, ausgebildet. Die künstliche Augenlinse ist bevorzugt eine Intraokularlinse.

Vorzugsweise ist vorgesehen, dass der optische Teil der multifokalen, künstlichen Augenlinse einen Durchmesser von größer als 6 mm aufweist. Dadurch erstreckt sich dieser optische Teil auch über eine große und/oder sehr weit zu öffnende Pupille eines Auges. Insbesondere ist dieser Durchmesser des optischen Teils größer als 6,5 mm.

Es kann vorgesehen sein, dass die Struktur der Perforationszonen zumindest einen um die optische Hauptachse umlaufenden Ring aufweist. Es kann zusätzlich oder anstatt dazu auch vorgesehen sein, dass die Struktur der Perforationszonen zumindest eine senkrecht zur optischen Hauptachse orientierte Linie mit Perforationszonen aufweist. Insbesondere ist vorgesehen, dass die Struktur eine um die optische Hauptachse umlaufende Sternform aufweist, die durch die Anordnung der Perforationszonen entsprechend gebildet ist. Damit kann wiederum individuellen Bedürfnissen bezüglich der erforderlichen Örtlichkeit der Perforationszonen mit den Medikamenten und/oder der gegebenenfalls örtlich fokussierteren Vorteilhaftigkeit einer größeren Menge eines Medikaments und/oder der örtlich fokussierteren Vorteilhaftigkeit von zumindest zwei unterschiedlichen Medikamenten Rechnung getragen werden.

Vorzugsweise weist die künstliche Augenlinse eine weitere Struktur auf, die als künstliche Aperturblende oder Beschriftung in dem optischen Teil ausgebildet ist. Diese weitere Struktur, die somit eine zur in der Haptik und/oder der Einfassung ausgebildeten ersten Struktur unterschiedliche Struktur darstellt, ist in diesem optischen Teil in einem radialen Abstand von größer oder gleich 3 mm zur optischen Hauptachse ausgebildet. Diese weitere Struktur ist insbesondere als Mikroperforation mit weiteren Perforationszonen in diesem optischen Teil ausgebildet. Es kann vorgesehen sein, dass diese weitere Struktur als Laserstruktur ausgebildet ist und somit mit einem Laser erzeugt ist. Diese weiteren Perforationszonen der weiteren Struktur können mit einem Farbstoff gefüllt sein. Durch eine derartige Ausgestaltung ist es dann einem Operateur einfach möglich, die Charakterisierung der Augenlinse bezüglich zum Beispiel ihrer optischen Parameterwerte und/oder bezüglich ihrer Orientierung im Auge, beispielsweise bei einer torischen multifokalen, künstlichen Augenlinse einfach zu erkennen. Es kann dazu auch vorgesehen sein, dass diese weiteren Perforationszonen einen Durchmesser von wenigen Mikrometern aufweisen und mit einem Ultrakurzpulslaser erzeugt sind. Durch eine derartige Ausgestaltung sind sie bezüglich der optischen Abbildungseigenschaften des optischen Teils nicht beeinträchtigend ausgebildet und ermöglichen dennoch das Einschreiben visuell auslesbarer Informationen innerhalb des optischen Teils, wie es oben dargelegt ist. So kann beispielsweise auch die Lage von Zylinderachsen, die refraktiven Parameter als auch eine Typenbezeichnung entsprechend als auslesbare Informationen in dem optischen Teil erzeugt werden. Das Auslesen kann dann beispielsweise mit einer Spaltlampe auch in der Nachsorge von Patienten sicher erfolgen. Insbesondere ist es auch ermöglicht, außerhalb eines photopischen Pupillendurchmessers und/oder eines mesopischen Pupillendurchmessers eine derartige Beschriftung vorzusehen.

Die Perforationszonen können hier beispielsweise auch als ringabschnittförmige Kavitätenkanäle oder auch als geschlossene Ringe in Form von Kavitätenkanälen ausgebildet sein und eine innere Einfärbung in Form einer Aperturblende als künstliche Pupille aufweisen, indem ein absorbierender, biokompatibler Farbstoff eingebracht ist. Diese absorbierenden Kavitätenkanäle können zur Unterdrückung von Streulicht unter extremen Sehbedingungen, eventuell bei diffraktiven Strukturen, eingesetzt sein beziehungsweise ausgebildet sein. Zusätzlich oder anstatt dazu kann auch ein Fluoreszenzfarbstoff für eine Beschriftung vorgesehen sein, wobei hier beispielsweise ein Infrarot-Farbstoff vorgesehen sein kann.

Eine Aperturblende kann alternativ oder zusätzlich zu einem Farbstoffe auch mittels eines unterschiedlichen Polymerisationsgrades des Werkstoffes der künstlichen Augenlinse durch Einwirkung eines Laserstrahls erzeugt werden, insbesondere durch Multiphotonen-Polymerisation.

In einer vorteilhaften Ausführungsform ist vorgesehen, dass diese Mikroperforation der weiteren Struktur derart ausgebildet ist, dass die Aperturblende abhängig vom einfallenden Licht in ihrer Öffnungsweite automatisch veränderbar ist. Damit wird auch innerhalb der künstlichen Augenlinse eine veränderbare Lichtdurchlässigkeit geschaffen und diesbezüglich auch eine künstliche Pupille bereitgestellt. Dies kann, wie bereits oben dargelegt, insbesondere durch spezifische Farbstoffe, die dann in dieser Mikroperforation eingebracht sind, erreicht werden.

Vorzugsweise ist vorgesehen, dass die Mikroperforation eine Vielzahl von Perforationszonen aufweist, die bezüglich ihres Abstands zueinander unterschiedlich angeordnet sind. Insbesondere kann hier eine statistische Verteilung zueinander ausgebildet sein. Die Vielzahl von Perforationszonen kann zusätzlich oder anstatt dazu auch bezüglich ihrer Lage zueinander unterschiedlich angeordnet sein, und auch hier insbesondere eine statistische Verteilung dieser Lage realisiert sein. Auch zusätzlich oder anstatt dazu kann vorgesehen sein, dass diese Vielzahl von Perforationszonen bezüglich ihrer Abmessungen zueinander unterschiedlich ausgebildet ist. Insbesondere können als Abmessung hier ein Zonendurchmesser der Perforationszone und/oder eine Zonentiefe einer Perforationszone entsprechend unterschiedlich gestaltet sein.

Insbesondere weist zumindest eine Mikroperforation zumindest eine Perforationszone auf, die ein Ringkanal ist, der somit insbesondere ein in sich geschlossener vollständig umlaufender Ringkanal ist. Dies ist insbesondere für die Ausgestaltung einer künstlichen Pupille mit insbesondere sich lichtabhängig radial verändernder Lichtdurchlässigkeit vorteilhaft.

Vorzugsweise ist vorgesehen, dass die Mikroperforation zumindest eine Perforationszone aufweist, die mit zumindest einem bezüglich des Absorptionsverhaltens wellenlängenselektiven beziehungsweise intensitätsabhängigen Farbstoff zumindest bereichsweise gefüllt ist.

Es kann auch vorgesehen sein, dass in zumindest eine Perforationszone dieser weiteren Struktur eine in radialer Richtung zur optischen Hauptachse bezüglich des Absorptionsverhaltens variierende Farbstoffzusammensetzung eingebracht ist, bei welcher mit zunehmender Intensität des einfallenden Lichts das Absorptionsverhalten in radialer Richtung zur optischen Hauptachse nach innen betrachtet zunimmt.

Es kann in einer weiteren Ausführungsform vorgesehen sein, dass der optische Teil der Augenlinse eine weitere Struktur aufweist, die als Mikroperforation zumindest umlaufend um die optische Hauptachse des optischen Teils ausgebildet ist. Diese weitere Struktur ist in dem optischen Teil in einem ersten Radiusintervall zwischen 1,5 mm und 2,5 mm zur optischen Hauptachse und/oder in einem zweiten Radiusintervall zwischen 3,0 mm und 4,0 mm zur optischen Hauptachse ausgebildet. Es kann vorgesehen sein, dass durch diese in radialer Richtung an individuellen Stellen ausgebildeten Strukturringe individuelle Informationsmöglichkeiten über die Augenlinse mitgeteilt werden können und andererseits zusätzliche Funktionalität der Augenlinse gegeben werden kann, insbesondere die Erzeugung einer künstlichen Pupille.

Es kann vorgesehen sein, dass auf zumindest einer Seite des optischen Teils ein diffraktives Element ausgebildet ist. Zumindest eine Seite kann auch sphärisch oder asphärisch ausgebildet sein. Ebenso kann zumindest eine Seite ein torisches Oberflächenprofil aufweisen. Ebenso können auf zumindest einer Seite des optischen Teils ringförmige Zonen ausgebildet sein, die als Fresnelzonen realisiert sein können. Auch eine Ausgestaltung derartiger ringförmiger Zonen dahingehend, dass eine solche ringförmige Zone eine Hauptunterzone und eine daran anschließende Phasenunterzone aufweist, kann vorgesehen sein. Bei einer derartigen Ausgestaltung ist eine Phasenunterzone nicht parallel zur optischen Hauptachse orientiert, sondern diesbezüglich geneigt dazu angeordnet und hat eine eigene Brechkraft, die zur Gesamtbrechkraft dieser ringförmigen Zone beiträgt. Eine derartige Phasenunterzone erzeugt eine optische Weglängendifferenz zwischen zwei an diese Phasenunterzone in radialer Richtung jeweils direkt anschließenden Hauptunterzonen. Die genannten Ausgestaltungen der zumindest einen Seite des optischen Teils können auch alleine oder in beliebigen Kombinationen ausgebildet sein.

Um neben der refraktiven Brechkraft der Augenlinse infolge ihrer Brechkraft und Formgebung zusätzliche diffraktive Eigenschaften einzubringen, ist es in vorteilhafter Ausführung auch vorgesehen, eine reguläre und somit gleichmäßige Anordnung dieser Mikrostruktur und somit der Perforationszonen und eine reguläre Durchmessererzeugung dieser Perforationszonen auszubilden.

Es kann auch vorgesehen sein, dass bei einer implantierten Augenlinse ein Nachstar als mögliche Komplikation nach einer Kataraktoperation auftreten kann, der durch die Proliferation von Epithelzellen hin zur hinteren Kapselsackmembran verursacht wird. Um dem zumindest lindernd entgegenzutreten, ist vorzugsweise vorgesehen, dass als Perforationszonen Mikrostrukturen zu denen auch Nanostrukturen zu zählen sind, zur Veränderung der Rauheit und Benetzbarkeit der Augenlinse außerhalb des optischen Teils ausgebildet sind, insbesondere als Laserstrukturen ausgebildet sind. Dadurch können Zelladhäsion und Proliferation beeinflusst werden. Es können somit Leitstrukturen geschaffen werden, die ein gerichtetes Zellwachstum begünstigen und somit die negativen Effekte diesbezüglich zumindest deutlich unterdrücken können. Insbesondere Nanostrukturen, welche durch eine Interferenzstrukturierung hergestellt werden können, bewirken eine Änderung in der Verteilung der fokalen Adhäsionsstellen der Zelle und beeinflussen unter anderem komplexe Mechanismen wie die Zellproliferation. Ein Aufbringen von dicht angeordneten Bohrungen und Semiperforationszonen als Sacklöcher außerhalb des optischen Teils ermöglicht darüber hinaus ein Anhaften der hinteren Kapselsackmembran. Ein Beispiel für eine solche Struktur kann eine Wabenstruktur sein, in welcher die als Mikrobohrungen ausgebildeten Perforationszonen angeordnet und ausgebildet sind.

Des Weiteren betrifft die Erfindung auch ein Verfahren zum Herstellen einer multifokalen, künstlichen Augenlinse gemäß der oben genannten Aspekte, bei welchem eine Struktur mit einer Laser-Vorrichtung erzeugt wird und ein gepulster Laserstrahl mit einer Pulslänge zwischen 100 fs und 20 ps, einer Wellenlänge zwischen 200 nm und 1.100 nm, einer Pulswiederholrate zwischen 1 kHz und 10 MHz, einem Fokusdurchmesser von kleiner als 5 µm und einer Leistungsdichte von größer als 10⁶ W/cm² erzeugt wird und auf den Werkstoff der künstlichen Augenlinse einwirkt. Vorzugsweise beträgt die Pulslänge 300 fs und die Wellenlänge vorzugsweise 1.060 nm oder 532 nm oder 355 nm. Durch eine disruptive Bearbeitung kann vorzugsweise eine Wellenlänge von 256 nm und bevorzugt von 213 nm für insbesondere ablative Bearbeitung vorgesehen sein.

Ein weiterer unabhängiger Aspekt der Erfindung betrifft eine künstliche Augenlinse, die einen optischen Teil aufweist, mittels welchem die optische Abbildungseigenschaft der künstlichen Augenlinse charakterisiert ist. Dieser optische Teil weist eine erste optische Seite und eine in Richtung der optischen Hauptachse dieser künstlichen Augenlinse betrachtet gegenüberliegende zweite optische Seite auf. Die künstliche Augenlinse weist eine Struktur auf, die als Mikroperforation ausgebildet ist und zumindest bereichsweise umlaufend um die optische Hauptachse des optischen Teils ausgebildet ist. Der optische Teil weist einen Durchmesser von größer als 6 mm, insbesondere größer oder gleich 6,5 mm, auf. Die Struktur ist als künstliche Aperturblende in dem optischen Teil in einem radialen Abstand von größer oder gleich 3 mm zur optischen Hauptachse als Mikroperoration in dem optischen Teil ausgebildet.

Ein weiterer unabhängiger Aspekt der Erfindung betrifft eine künstliche Augenlinse, die einen optischen Teil aufweist, mittels welchem die optische Abbildungseigenschaft der künstlichen Augenlinse charakterisiert ist. Dieser optische Teil weist eine erste optische Seite und eine in Richtung der optischen Hauptachse dieser künstlichen Augenlinse betrachtet gegenüberliegende zweite optische Seite auf. Die multifokale, künstliche Augenlinse weist des Weiteren eine Haptik auf. Mit der Haptik ist die Augenlinse positionell im Auge gehalten. Die multifokale, künstliche Augenlinse weist zusätzlich oder anstatt der Haptik eine den optischen Teil zumindest bereichsweise umgebende und zur Haptik unterschiedliche Einfassung auf. Diese Einfassung ist dann weder Bestandteil des optischen Teils noch ist sie Bestandteil einer Haptik, wenn eine derartige Haptik vorhanden ist. Die multifokale, künstliche Augenlinse weist eine Struktur auf, die zumindest eine Vertiefung aufweist. Die Struktur ist als Mikroperforation mit einer Vielzahl von einzelnen und separaten Perforationszonen ausgebildet. Die Perforationszonen sind als Sacklöcher ausgebildet. Die Perorationszonen bilden ein Netzgebilde. Das Netzgebilde ist insbesondere eine Wabenform.

Ein weiterer unabhängiger Aspekt der Erfindung betrifft eine künstliche Augenlinse, die einen optischen Teil aufweist, mittels welchem die optische Abbildungseigenschaft der künstlichen Augenlinse charakterisiert ist. Dieser optische Teil weist eine erste optische Seite und eine in Richtung der optischen Hauptachse dieser künstlichen Augenlinse betrachtet gegenüberliegende zweite optische Seite auf. Die künstliche Augenlinse weist eine Struktur auf, die als Mikroperforation ausgebildet ist und zumindest bereichsweise umlaufend um die optische Hauptachse des optischen Teils ausgebildet ist. Die Struktur ist in dem optischen Teil ausgebildet. Die Struktur ist in dem optischen Teil in einem ersten Radiusintervall zwischen 1,5 mm und 2,5 mm zur optischen Hauptachse und/oder ist in einem zweiten Radiusintervall zwischen 3,0 mm und 4,0 mm zur optischen Hauptachse ausgebildet.

Ausführungen des ersten unabhängigen Aspekts der Erfindung sind als vorteilhafte Ausführungen der weiteren unabhängigen Aspekte anzusehen. Entsprechendes gilt für das Herstellungsverfahren.

Weitere Merkmale der Erfindung ergeben sich aus den Ansprüchen, den Figuren und der Figurenbeschreibung. Die vorstehend in der Beschreibung genannten Merkmale und Merkmalskombinationen, sowie die nachfolgend in der Figurenbeschreibung genannten und/oder in den Figuren alleine gezeigten Merkmale und Merkmalskombinationen sind nicht nur in der jeweils angegebenen Kombination, sondern auch in anderen Kombinationen verwendbar, ohne den Rahmen der Erfindung zu verlassen. Es sind somit auch Ausführungen von der Erfindung als umfasst und offenbart anzusehen, die in den Figuren nicht explizit gezeigt und erläutert sind, jedoch durch separierte Merkmalskombinationen aus den erläuterten Ausführungen hervorgehen und erzeugbar sind. Es sind auch Ausführungen und Merkmalskombinationen als offenbart anzusehen, die somit nicht alle Merkmale eines ursprünglich formulierten unabhängigen Anspruchs aufweisen. Es sind darüber hinaus Ausführungen und Merkmalskombinationen, insbesondere durch die oben dargelegten Ausführungen, als offenbart anzusehen, die über die in den Rückbezügen der Ansprüche dargelegten Merkmalskombinationen hinausgehen oder abweichen.

Die in den Unterlagen angegebenen konkreten Werte von Parametern und Angaben zu Verhältnissen von Parametern bzw. Parameterwerten zur Definition von Ausführungsbeispielen der Augenlinse sind auch im Rahmen von Abweichungen, beispielsweise aufgrund von Messfehlern, Systemfehlern, DIN-Toleranzen etc. als vom Rahmen der Erfindung mit umfasst anzusehen, wodurch auch Erläuterungen, die sich auf im Wesentlichen entsprechende Werte und Angaben beziehen, darunter zu verstehen sind.

### Kurze Beschreibung der Zeichnungen

Ausführungsbeispiele der Erfindung werden nachfolgend anhand schematischer Zeichnungen näher erläutert. Es zeigen:
- Fig. 1: eine Draufsicht auf ein erstes Ausführungsbeispiel einer erfindungsgemäßen künstlichen Augenlinse in schematischer Darstellung;
- Fig. 2: eine Draufsicht auf ein zweites Ausführungsbeispiel einer erfindungsgemäßen künstlichen Augenlinse in schematischer Darstellung;
- Fig. 3: eine Draufsicht auf ein drittes Ausführungsbeispiel einer erfindungsgemäßen künstlichen Augenlinse in schematischer Darstellung; und
- Fig. 4: eine vereinfachte schematische Darstellung einer Laser-Vorrichtung zum Herstellen einer Strukturierung an einer Augenlinse gemäß Fig. 1 bis 3.

### Bevorzugte Ausführungen der Erfindung

In den Figuren sind gleiche oder funktionsgleiche Elemente mit den gleichen Bezugszeichen versehen.

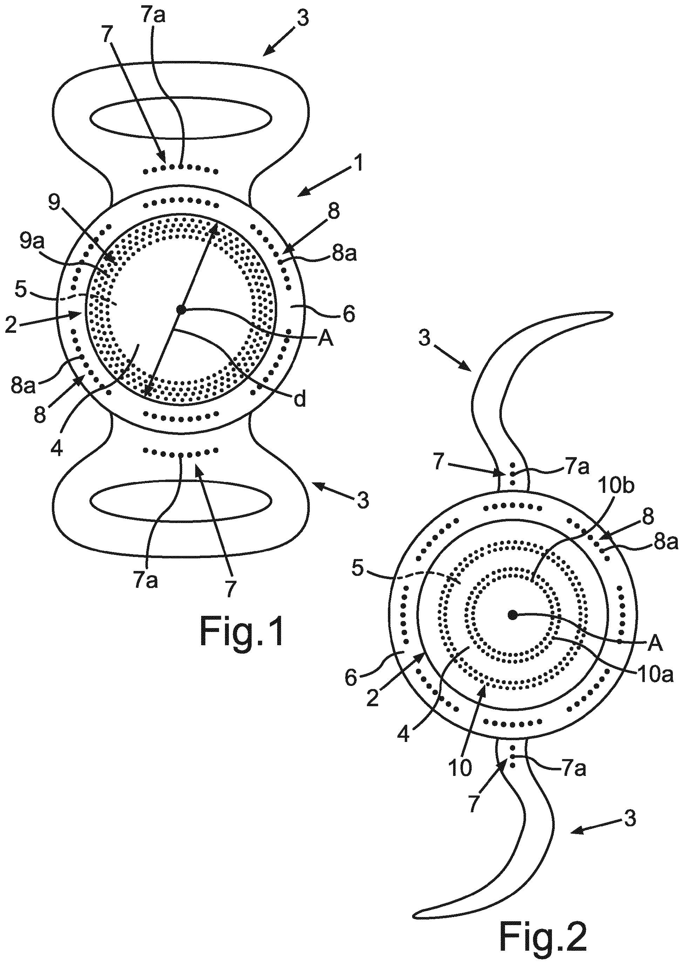

In Fig. 1 ist in einer Draufsicht ein erstes Ausführungsbeispiel einer multifokalen, künstlichen Augenlinse 1 gezeigt, die hier eine Intraokularlinse ist. Die künstliche Augenlinse 1 weist einen optischen Teil 2 und daran anschließend eine Haptik 3 auf. Die künstliche Augenlinse 1 ist faltbar und über einen Kleinschnitt in ein Auge einführbar. Der optische Teil 2, der wesentlich für die optische Abbildungseigenschaft der künstlichen Augenlinse 1 ist, weist eine optische Hauptachse A auf, die senkrecht zur Figurenebene orientiert ist und insbesondere senkrecht auf einer Hauptebene des optischen Teils 2 steht. Der optische Teil 2 weist darüber hinaus in Richtung dieser optischen Hauptachse A betrachtet eine erste optische Fläche bzw. optische Seite 4 auf, die eine Vorderseite sein kann, und weist gegenüberliegend eine zweite optische Fläche bzw. optische Seite 5 auf, die eine Rückseite sein kann. Die Vorderseite ist im implantierten Zustand der Augenlinse 1 im Auge der Hornhaut zugewandt, wohingegen die Rückseite dieser Hornhaut abgewandt ist.

Die künstliche Augenlinse 1 kann zusätzlich zur Haptik 3 oder anstatt dazu eine Einfassung 6 aufweisen. Die Einfassung 6 ist nicht Bestandteil des optischen Teils 2 und trägt somit nicht zur optischen Abbildungseigenschaft der künstlichen Augenlinse 1 und somit auch nicht des optischen Teils 2 bei. Selbiges gilt für die Haptik 3. Die Einfassung 6 ist hier als vollständig umlaufender Ring ausgebildet. Die Einfassung 6 kann aber auch nur als Ringabschnitt ausgebildet sein, und beispielsweise nur im Bereich zwischen einer Haptik 3 und dem optischen Teil 2 ausgebildet sein, beispielsweise als Brücke bzw. Verbinder zwischen diesen Komponenten.

In Fig. 2 ist in einer Draufsicht ein weiteres Ausführungsbeispiel einer als Intraokularlinse ausgebildeten multifokalen, künstlichen Augenlinse 1 gezeigt. Sie unterscheidet sich von der Ausführung in Fig. 1 durch die unterschiedliche Haptik 3. Mittels der Haptik 3 ist auch hier die Augenlinse 1 im Auge, insbesondere im Kapselsack, gehalten.

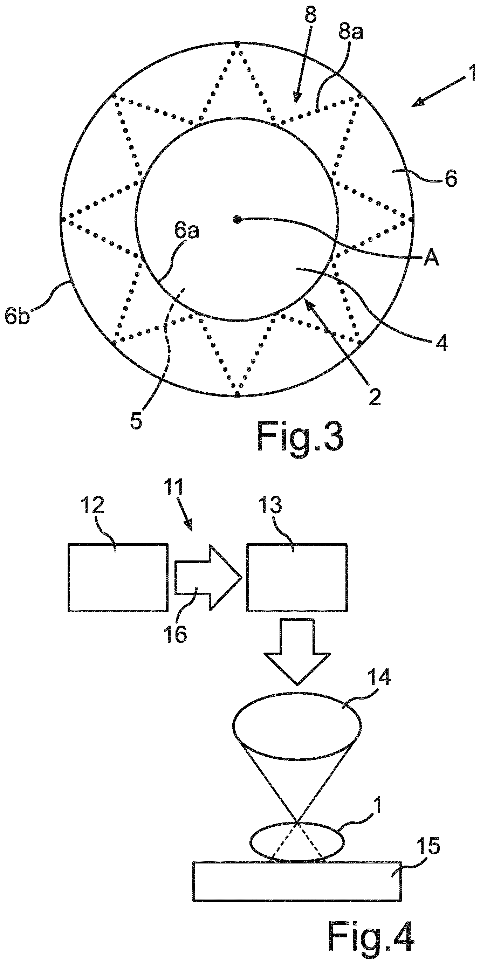

In Fig. 3 ist ein weiteres Ausführungsbeispiel einer Augenlinse 1 in einer Draufsicht gezeigt. Hier ist die Einfassung 6 auch vorzugsweise als umlaufender Ring ausgebildet. Die Haptik kann hier auch entfallen und die Funktion durch die Einfassung 6 mit übernommen werden. Es kann aber auch vorgesehen sein, dass Ringabschnitte dieses Rings um den optischen Teil 2 eine nicht vollständig umlaufende Einfassung 6 bilden und andere Ringabschnitte eine Haptik bilden.

Die optischen Seiten 4 und 5 sind in den Ausführungen vorzugsweise uneben, insbesondere konvex, gekrümmt. Insbesondere ist auf zumindest einer optischen Seite 4, 5 auf dieser konvexen, insbesondere sphärischen oder asphärischen, Grundform ein diffraktives Profil und/oder ein torisches Profil ausgebildet.

Grundsätzlich können auch anderweitig geformte und ausgestaltete Haptiken 3 und/oder Einfassungen 6 vorgesehen sein.

Die multifokale, künstliche Augenlinse 1, wie sie in Fig. 1 und Fig. 2 in unterschiedlichen Ausführungsbeispielen gezeigt ist, weisen eine Struktur 7 der Haptik 3, wenn diese vorhanden ist, und/oder eine Struktur 8 der Einfassung 6, wenn diese vorhanden ist, auf. Es sei an dieser Stelle dargelegt, dass sowohl die örtlichen Positionen als auch die geometrischen Ausgestaltungen der Strukturen 7 und 8 lediglich symbolhaft und zur Verdeutlichung dieser Strukturen zu verstehen sind und diesbezüglich keine abschließende Spezifikation darstellen. Die Struktur 7 ist als Mikroperforation mit einer Vielzahl von Perforationszonen 7a ausgebildet. Die Perforationszonen 7a sind insbesondere als Sacklöcher ausgebildet und als Mikrobohrungen ausgebildet. Dazu sind sie als Laserstruktur gestaltet und mit einer Laser-Vorrichtung erzeugt.

Zumindest einige dieser Perforationszonen 7a sind als Medikamentendepots ausgebildet und somit bereichsweise mit zumindest einem Medikament befüllt. Zusätzlich oder anstatt dazu kann auch vorgesehen sein, dass die Struktur 8 eine Mikroperforation ist und eine Vielzahl von Perforationszonen 8a aufweist. In Fig. 1 ist der Übersichtlichkeit dienend nur an einer Stelle eine derartige Darstellung von Perforationszonen 8a angedeutet. Auch diese Perforationszonen 8a sind vorzugsweise als Sacklöcher ausgebildet und mit einer Laser-Vorrichtung erzeugt. Auch hier sind zumindest einige der Perforationszonen 8a als Medikamentendepots ausgebildet und mit zumindest einem Medikament zumindest bereichsweise gefüllt. Die Perforationszonen 7a und/oder 8a können in einer oder in mehreren Linien ausgebildet sein. Sie können geometrisch gleich oder unterschiedlich zueinander ausgebildet sein. Sie können regulär angeordnet sein oder statistisch verteilt angeordnet sein.

Es kann vorgesehen sein, dass die Struktur 8 zumindest einen um die optische Hauptachse A umlaufenden Ring aufweist. Zusätzlich oder anstatt dazu kann die Struktur 8 einen radial zur optischen Hauptachse A orientierten Strukturbereich aufweisen. Auch anderweitige geometrische Ausgestaltungen von Perforationszonen können bei der Struktur 7 und/oder der Struktur 8 ausgebildet sein.

In einem Ausführungsbeispiel kann, insbesondere unabhängig von der örtlichen und geometrischen Ausgestaltung einer Struktur 7 und/oder 8, der optische Teil 2 einen Durchmesser d aufweisen, der größer als 6 mm, insbesondere größer als 6,5 mm, ist.

Der optische Teil 2 kann, ebenfalls insbesondere durch die Laser-Vorrichtung als Laserstruktur erzeugt, eine Struktur 9 aufweisen, die eine weitere Struktur 9 sein kann, die als künstliche Aperturblende ausgebildet ist. Insbesondere ist diese künstliche Aperturblende in einem radialen Abstand von größer oder gleich 3 mm zur optischen Hauptachse A in dem optischen Teil 2 ausgebildet, wenn dieser optische Teil 2 einen Durchmesser von größer als 6 mm aufweist. Auch diese weitere Struktur 9 ist insbesondere als Mikroperforation ausgebildet. Diese weitere Struktur 9 ist insbesondere mit zumindest einem Farbstoff gefüllt. Vorzugweise ist diese Mikroperforation der weiteren Struktur 9 derart ausgebildet, dass die Aperturblende abhängig von einfallendem Licht in ihrer Öffnungsweite automatisch veränderbar ist. Eine künstliche Pupille ist dadurch in der künstlichen Augenlinse 1 selbst geschaffen.

Diese Mikroperforation der weiteren Struktur 9 weist insbesondere eine Vielzahl von Perforationszonen 9a auf, die bezüglich ihres Abstands zueinander unterschiedlich angeordnet sind, insbesondere statistisch verteilt zueinander angeordnet sind, und/oder bezüglich ihrer Lage zueinander unterschiedlich angeordnet sind, insbesondere statistisch verteilt zueinander angeordnet sind, und/oder bezüglich ihrer Abmessungen, insbesondere eines Perforationszonendurchmessers und/oder einer Perforationszonentiefe, zueinander unterschiedlich ausgebildet sind.

Es kann auch vorgesehen sein, dass diese weitere Struktur 9 zumindest einen, vollständig um die optische Hauptachse A umlaufenden Ring aufweist, insbesondere zwei derartig um die optische Hauptachse vollständig umlaufende Ringe aufweist, die in radialer Richtung zur optischen Hauptachse A beabstandet zueinander ausgebildet sind. Es kann vorgesehen sein, dass insbesondere diese weitere Struktur 9 in einem ersten Radiusintervall zwischen 1,5 mm und 2,5 mm zur optischen Hauptachse A und/oder in einem zweiten Radiusintervall zwischen 3,0 mm und 4,0 mm zur optischen Hauptachse jeweils einen Ring aus Mikroperforationen und somit Perforationszonen, die als Mikrobohrungen ausgebildet sind, aufweist. Diese Ausgestaltung kann jedoch auch durch eine zur weiteren Struktur 9 wiederum unterschiedliche diesbezüglich nochmals weitere Struktur ausgebildet sein.

Die zu Fig. 1 erläuterten und unterschiedlichen Ausführungsbeispiele sind auch für die Ausgestaltung gemäß Fig. 2 und Fig. 3 möglich. In Fig. 2 ist die in Fig. 1 erläuterte und dort der Übersichtlichkeit dienend nicht eingezeichnete nochmals weitere Struktur 10 mit den bei den spezifischen Radiusintervallen dargelegten Perforationsringen schematisch gezeigt. Diese Ringe 10a und 10b bei den Radiusintervallen zwischen 1,5 mm und 2,5 mm sowie zwischen 3,0 mm und 4,0 mm sind hier der Übersichtlichkeit dienend ebenfalls nur symbolisch dargestellt.

In Fig. 3 ist eine spezifische Geometrie der Struktur 8 gezeigt, die hier eine Sternform darstellt. Diese Sternform erstreckt sich an einem inneren Rand 6a der Einfassung 6 bis zu einem äußeren Rand 6b dieser Einfassung 6. Die Perforationszonen 8a sind hier ebenfalls symbolhaft gezeigt. Eine derartige Sternform der Struktur 8 kann auch bei den Ausführungsbeispielen in Fig. 1 und Fig. 2 vorgesehen sein. Zur Verdeutlichung dieser Sternform ist die Einfassung radial vergrößert im Vergleich zum optischen Teil 2 dargestellt.

Bei den Ausführungen kann vorgesehen sein, dass einige der Perforationszonen 7a und/oder 8a in Richtung der optischen Seite 4 des optischen Teils 2 hin offen ausgebildet sind oder die Perforationszonen 7a und 8a nur in Richtung der optischen Seite 5 des optischen Teils 2 hin offen ausgebildet sind. Es kann auch vorgesehen sein, dass einige der Perforationszonen 7a und/oder 8a in Richtung der optischen Seite 4 hin offen ausgebildet sind und einige der Perforationszonen 7a und 8a in Richtung der optischen Seite 5 hin offen ausgebildet sind. Insbesondere eignen sich diese Alternativen, wenn diese Perforationszonen 7a und 8a als Sacklöcher ausgebildet sind. Eine wiederum diesbezüglich individuelle Gestalt der Perforationszonen und ihrer Öffnungen ist hier ermöglicht, sodass auch hier individuell örtlich eine spezifische Abgabe des Medikaments und/oder der Medikamente ermöglicht ist.

In Fig. 4 ist in einer schematischen Darstellung eine Laser-Vorrichtung 11 gezeigt, welche zum Herstellen einer multifokalen, künstlichen Augenlinse 1 ausgebildet ist. Insbesondere ist mit dieser Laser-Vorrichtung 11 die Erzeugung einer Struktur 7 und/oder einer Struktur 8 und/oder einer Struktur 9 und/oder einer Struktur 10 ermöglicht. Die Laser-Vorrichtung 11 weist zumindest einen Laser 12 auf, welcher ein Ultrakurzpulslaser ist. Diese Laser-Vorrichtung 11 weist einen insbesondere dreidimensional einstellbaren Scanner 13 auf, mittels welchem der gepulste Laserstrahl des Lasers 12 einstellbar ist. Des Weiteren weist die Laser-Vorrichtung 11 eine Fokussieroptik 14 auf, die im Strahlengang dem Scanner 13 nachrangig angeordnet ist. Die Laser-Vorrichtung 11 weist darüber hinaus eine Aufnahme 15 auf, auf welcher die künstliche Augenlinse 1 aufgebracht wird, um dann die gewünschte Strukturierung mit dem durch die Fokussieroptik 14 fokussierten Laserstrahl 16 einwirken lassen zu können. Der Laserstrahl 16 mit seinen Laserpulsen wird insbesondere mit einer Pulslänge zwischen 100 fs und 20 ps, insbesondere einer Wellenlänge zwischen 200 nm und 1.100 nm, insbesondere einer Pulswiederholrate zwischen 1 kHz und 10 MHz, insbesondere einem Fokusdurchmesser kleiner als 5 µm und insbesondere einer Leistungsdichte von größer als 10⁸W/cm² erzeugt. Es wird hier insbesondere eine Multiphotonenabsorption ermöglicht. Die Fokussieroptik 14 kann eine numerische Apertur von größer als 0,1, vorzugsweise größer als 0,3 und insbesondere größer als 0,5 aufweisen. Mit der Laser-Vorrichtung 11 ist auch das Erzeugen von Fokusdurchmessern von kleiner als 5 µm, insbesondere von kleiner als 2 µm, möglich. Dabei ist eine Leistungsdichte des fokussierten Laserstrahls von größer als 10¹⁰ W/cm² sinnvoll, um einen optischen Durchbruch (Photodisruption) des Polymerwerkstoffes der künstlichen Augenlinse zu erreichen, wenn beispielsweise keine lineare Absorption des Polymerwerkstoffes diesen Effekt unterstützt. Um lediglich eine nicht-lineare Wechselwirkung im Polymerwerkstoff der künstlichen Augenlinse 1 zu erzielen, ist auch eine Leistungsdichte von kleiner als 10¹⁰ W/cm² vorgesehen, die nicht zur Photodisruption führt, jedoch optische und/oder mechanische beziehungsweise damit einhergehende auch hygroskopische Werkstoffeigenschaften ändern kann. Um eine hohe Bearbeitungseffizienz der künstlichen Augenlinse sicherzustellen, ist eine Repetitionsrate der ultrakurzen Laserpulse des Laserstrahls 16 im Bereich von 1 kHz bis 10 MHz vorteilhaft. Dabei werden Pulsenergien im sub-µJ-Bereich benutzt. Insbesondere bei einer Repetitionsrate von größer als 1 MHz ist aufgrund kumulativer Wechselwirkungseffekte auch eine Pulsenergie von kleiner als 1 µJ vorgesehen.

Insbesondere die Struktur 7 und/oder die Struktur 8 können zusätzlich zu der Erläuterung der Ausgestaltungen zumindest bereichsweise als Medikamentendepots auch insbesondere in Mikrohüllen oder Nanohüllen und insbesondere in Form von zumindest bereichsweise um die optische Hauptachse A umlaufenden Ringen ausgebildet sein. Dadurch ist eine Veränderung der Rauheit und Benetzbarkeit der künstlichen Augenlinse 1 in diesen Bereichen möglich. Dies ist vorteilhaft, da somit das Auftreten von Nachstar vermindert werden kann beziehungsweise gezielt gerichtet ermöglicht ist.

Auch können insbesondere bei der Struktur 8 individuelle Perforationszonen vorgesehen und im Randbereich ausgebildet sein, um ein besseres Anhaften mit dem Kapselsack zu ermöglichen. Dadurch wird die positionelle Anordnung der Augenlinse 1 in dem Kapselsack ebenfalls verbessert. Die Anordnung kann nicht nur hier beispielsweise als Wabenstruktur ausgebildet sein, sodass die diesbezüglich vorgesehenen Perforationszonen 8a entsprechend zueinander ausgebildet sind. Diese Ausgestaltungen können zusätzlich und/oder optional zu den bereits vorstehend erläuterten Ausgestaltungen realisiert sein.

## Patentansprüche

1. Künstliche Augenlinse (1) mit einem optischen Teil (2), der eine in Richtung einer optischen Hauptachse (A) der künstlichen Augenlinse (1) betrachtet erste optische Seite (4) und eine gegenüberliegende zweite optische Seite (5) aufweist, und eine Haptik (3) aufweist, **dadurch gekennzeichnet, dass** in einer den optischen Teil (2) zumindest bereichsweise umgebend und zur Haptik (3) unterschiedlichen Einfassung (6) der künstlichen Augenlinse (1) eine Struktur mit zumindest einer Vertiefung ausgebildet ist, wobei
die Struktur (7, 8) als Mikroperforation mit einer Vielzahl von Perforationszonen (7a, 8a) ausgebildet ist und zumindest einige Perforationszonen (7a, 8a) zur Erzeugung eines Medikamentendepots mit zumindest einem Medikament zumindest bereichsweise gefüllt sind.

2. Künstliche Augenlinse (1) nach Anspruch 1,
**dadurch gekennzeichnet, dass**
die mit zumindest einem Medikament zumindest bereichsweise gefüllten Perforationszonen (7a, 8a) als Sacklöcher ausgebildet sind.

3. Künstliche Augenlinse (1) nach einem der vorhergehenden Ansprüche,
**dadurch gekennzeichnet, dass**
der optische Teil (2) einen Durchmesser (d) größer 6 mm, insbesondere größer 6,5 mm aufweist.

4. Künstliche Augenlinse (1) nach einem der vorhergehenden Ansprüche,
**dadurch gekennzeichnet, dass**
eine weitere Struktur (9) als künstliche Aperturblende in dem optischen Teil (2) in einem radialen Abstand von größer oder gleich 3 mm zur optischen Hauptachse (A) als Mikroperforation in dem optischen Teil (2) ausgebildet ist.

5. Künstliche Augenlinse (1) nach Anspruch 4,
**dadurch gekennzeichnet, dass**
die Mikroperforation derart ausgebildet ist, dass die Aperturblende abhängig vom einfallenden Licht in ihrer Öffnungsweite automatisch veränderbar ist.

6. Künstliche Augenlinse (1) nach Anspruch 4 oder 5,
**dadurch gekennzeichnet, dass**
die Mikroperforation eine Vielzahl von Perforationszonen (9a) aufweist, die bezüglich ihres Abstandes zueinander unterschiedlich angeordnet sind, insbesondere statistisch verteilt zueinander angeordnet sind, und/oder bezüglich ihrer Lage zueinander unterschiedlich angeordnet sind, insbesondere statistisch verteilt zueinander angeordnet sind, und/oder bezüglich ihren Abmessungen, insbesondere einem Zonendurchmesser und/oder einer Zonentiefe, zueinander unterschiedlich ausgebildet sind.

7. Künstliche Augenlinse (1) nach einem der Ansprüche 4 bis 6,
**dadurch gekennzeichnet, dass**
die Mikroperforation zumindest eine Perforationszone (9a) aufweist, die ein Ringkanal ist.

8. Künstliche Augenlinse (1) nach einem der Ansprüche 4 bis 7,
**dadurch gekennzeichnet, dass**
die Mikroperforation zumindest eine Perforationszone (9a) aufweist, die mit zumindest einem bezüglich des Absorptionsverhaltens wellenlängenselektiven Farbstoff zumindest bereichsweise gefüllt sind.

9. Künstliche Augenlinse (1) nach Anspruch 7 und 8,
**dadurch gekennzeichnet, dass**
in zumindest eine Perforationszone (9a) eine in radialer Richtung zur optischen Hauptachse (A) bezüglich des Absorptionsverhaltens variierende Farbstoffzusammensetzung eingebracht ist, bei welcher mit zunehmender Intensität des einfallenden Lichts das Absorptionsverhalten in radialer Richtung zur optischen Hauptachse (A) nach innen betrachtet zunimmt.

10. Künstliche Augenlinse (1) nach einem der vorhergehenden Ansprüche,
**dadurch gekennzeichnet, dass**
der optische Teil (2) eine weitere Struktur (10) aufweist, die als Mikroperforation und zumindest bereichsweise umlaufend um die optische Hauptachse (A) des optischen Teils (2) ausgebildet ist, wobei die weitere Struktur (10) in dem optischen Teil (2) in einem ersten Radiusintervall zwischen 1,5 mm und 2,5 mm zur optischen Hauptachse (A) und/oder in einem zweiten Radiusintervall zwischen 3,0 mm und 4,0 mm zur optischen Hauptachse (A) ausgebildet ist.

11. Künstliche Augenlinse (1) nach einem der vorhergehenden Ansprüche,
**dadurch gekennzeichnet, dass**
die Struktur (7, 8) und/oder eine weitere Struktur (9, 10) als Laserstruktur ausgebildet ist bzw. sind.

12. Verfahren zum Herstellen einer künstlichen Augenlinse (1) nach einem der vorhergehenden Ansprüche, bei welchem zumindest eine Struktur (7, 8, 9, 10) mit einer Laser-Vorrichtung (11) erzeugt wird, und ein gepulster Laserstrahl mit einer Pulslänge zwischen 100 fs und 20 ps, einer Wellenlänge zwischen 200 nm und 1100 nm, einer Pulswiederholrate zwischen 1 kHz und 10 Mhz, einem Fokusdurchmesser von kleiner als 5 µm und einer Leistungsdichte von größer als 10⁸ W/cm² erzeugt wird und auf das Material der Augenlinse (1) einwirkt.

## Claims

1. Artificial eye lens (1) comprising an optical part (2), which has a first optical side (4) as viewed in a direction of an optical principal axis (A) of the artificial eye lens (1) and an opposite second optical side (5), and has a haptic arrangement (3), **characterized in that** a structure with at least one depression is formed in a surround (6) of the artificial eye lens (1) that surrounds the optical part (2) at least in certain areas and that differs from the haptic arrangement (3), wherein
the structure (7, 8) is formed as a micro-perforation with a multiplicity of perforation zones (7a, 8a) and at least some perforation zones (7a, 8a) are filled at least in certain areas with at least one medicament for the purposes of producing a medicament repository.

2. Artificial eye lens (1) according to Claim 1, **characterized in that**
the perforation zones (7a, 8a) that are filled at least in certain areas with at least one medicament are formed as blind holes.

3. Artificial eye lens (1) according to either of the preceding claims,
**characterized in that**
the optical part (2) has a diameter (d) of greater than 6 mm, in particular greater than 6.5 mm.

4. Artificial eye lens (1) according to any one of the preceding claims,
**characterized in that**
a further structure (9) is formed as an artificial aperture stop in the optical part (2) as a micro-perforation in the optical part (2) at a radial distance of greater than or equal to 3 mm from the optical principal axis (A).

5. Artificial eye lens (1) according to Claim 4, **characterized in that**
the micro-perforation is formed in such a way that the aperture stop is automatically modifiable in terms of its opening width as a function of the incident light.

6. Artificial eye lens (1) according to Claim 4 or 5, **characterized in that**
the micro-perforation has a multiplicity of perforation zones (9a), which are disposed differently with respect to one another in terms of their spacing, which more particularly are disposed with a statistical distribution with respect to one another, and/or which are disposed differently with respect to one another in terms of their position, which more particularly are disposed with a statistical distribution with respect to one another, and/or which have a different embodiment with respect to one another in terms of their dimensions, in particular a zone diameter and/or a zone depth.

7. Artificial eye lens (1) according to any one of Claims 4 to 6,
**characterized in that**
the micro-perforation has at least one perforation zone (9a) which is a ring channel.

8. Artificial eye lens (1) according to any one of Claims 4 to 7,
**characterized in that**
the micro-perforation has at least one perforation zone (9a) which is filled at least in certain areas with at least one dye that is wavelength selective in respect of the absorption behaviour.

9. Artificial eye lens (1) according to Claim 7 and 8, **characterized in that**
a dye composition that varies in respect of the absorption behaviour in a radial direction with respect to the optical principal axis (A) is introduced into at least one perforation zone (9a), the absorption behaviour of said dye composition, when considered inwardly, increasing in the radial direction with respect to the optical principal axis (A) with increasing intensity of the incident light.

10. Artificial eye lens (1) according to any one of the preceding claims,
**characterized in that**
the optical part (2) comprises a further structure (10) which is formed as a micro-perforation and as surrounding the optical principal axis (A) of the optical part (2) at least in certain areas, wherein the further structure (10) is formed in the optical part (2) in a first radius interval of between 1.5 mm and 2.5 mm from the optical principal axis (A) and/or in a second radius interval of between 3.0 mm and 4.0 mm from the optical principal axis (A) .

11. Artificial eye lens (1) according to any one of the preceding claims,
**characterized in that**
the structure (7, 8) and/or a further structure (9, 10) are/is formed as a laser structure.

12. Method for producing an artificial eye lens (1) according to any one of the preceding claims, in which at least one structure (7, 8, 9, 10) is produced with a laser apparatus (11), and a pulsed laser beam having a pulse length of between 100 fs and 20 ps, a wavelength of between 200 nm and 1100 nm, a pulse repetition rate of between 1 kHz and 10 MHz, a focus diameter of less than 5 µm, and a power density of greater than 10⁸ W/cm² is produced and acts on the material of the eye lens (1).

## Revendications

1. Lentille oculaire artificielle (1) comportant une partie optique (2) qui comporte un premier côté optique (4) et un deuxième côté optique opposé (5) lorsqu'elle est vue dans la direction d'un axe optique principal (A) de la lentille oculaire artificielle (1) et une haptique (3), **caractérisée en ce qu'**une structure pourvue d'au moins un évidement est formée dans un contour (6) de la lentille oculaire artificielle (1) qui entoure la partie optique (2) au moins par zones et qui est différent de l'haptique (3),
la structure (7, 8) étant réalisée sous forme de micro-perforation pourvue d'un grand nombre de zones de perforation (7a, 8a) et au moins certaines zones de perforation (7a, 8a) étant remplies au moins par zones d'au moins un médicament afin de générer un dépôt de médicament.

2. Lentille oculaire artificielle (1) selon la revendication 1,
**caractérisée en ce que**
les zones de perforation (7a, 8a) qui sont remplies au moins par zones d'au moins un médicament sont réalisées sous forme de trous borgnes.

3. Lentille oculaire artificielle (1) selon l'une des revendications précédentes,
**caractérisée en ce que**
la partie optique (2) a un diamètre (d) supérieur à 6 mm, en particulier supérieur à 6,5 mm.

4. Lentille oculaire artificielle (1) selon l'une des revendications précédentes,
**caractérisée en ce que**
une autre structure (9) est réalisée sous la forme d'un diaphragme d'ouverture artificiel dans la partie optique (2) à une distance radiale supérieure ou égale à 3 mm de l'axe optique principal (A) sous forme de microperforation dans la partie optique (2).

5. Lentille oculaire artificielle (1) selon la revendication 4,
**caractérisée en ce que**
la microperforation est conçue de telle sorte que le diaphragme d'ouverture peut être modifié automatiquement, en termes de largeur d'ouverture, en fonction de la lumière incidente.

6. Lentille oculaire artificielle (1) selon la revendication 4 ou 5,
**caractérisée en ce que**
la microperforation comporte un grand nombre de zones de perforation (9a) qui sont disposées différemment en termes de distance les unes par rapport aux autres, en particulier disposées de manière statistiquement répartie les unes par rapport aux autres et/ou sont disposées différemment en termes de position les unes par rapport aux autres, en particulier, sont disposées de manière statistiquement répartie les unes par rapport aux autres, et/ou sont conçues différemment les unes des autres en termes de dimensions, notamment de diamètre de zone et/ou de profondeur de zone.

7. Lentille oculaire artificielle (1) selon l'une des revendications 4 à 6,
**caractérisée en ce que**
la microperforation comporte au moins une zone de perforation (9a) qui est un canal annulaire.

8. Lentille oculaire artificielle (1) selon l'une des revendications 4 à 7,
**caractérisée en ce que**
la microperforation comporte au moins une zone de perforation (9a) qui est remplie au moins par zones d'au moins un colorant sélectif en fonction de la longueur d'onde en ce qui concerne le comportement d'absorption.

9. Lentille oculaire artificielle (1) selon les revendications 7 et 8,
**caractérisée en ce que**
une composition colorante qui varie dans la direction radiale par rapport à l'axe optique principal (A) en ce qui concerne le comportement d'absorption est introduite dans au moins une zone de perforation (9a), composition colorante pour laquelle le comportement d'absorption augmente dans la direction radiale par rapport à l'axe optique principal (A), dans une vue vers l'intérieur, à mesure que l'intensité de la lumière incidente augmente.

10. Lentille oculaire artificielle (1) selon l'une des revendications précédentes,
**caractérisée en ce que**
la partie optique (2) comporte une autre structure (10) qui est réalisée sous la forme de microperforation et qui est conçue pour s'étendre au moins par zones circonférentiellement autour de l'axe optique principal (A) de la partie optique (2), l'autre structure (10) dans la partie optique (2) étant formée dans un premier intervalle de rayon compris entre 1,5 mm et 2,5 mm par rapport à l'axe optique principal (A) et/ou dans un deuxième intervalle de rayon compris entre 3,0 mm et 4,0 mm par rapport à l'axe optique principal (A).

11. Lentille oculaire artificielle (1) selon l'une des revendications précédentes,
**caractérisée en ce que**
la structure (7, 8) et/ou une autre structure (9, 10) sont réalisées sous la forme d'une structure laser.

12. Procédé de fabrication d'une lentille oculaire artificielle (1) selon l'une des revendications précédentes, procédé dans lequel au moins une structure (7, 8, 9, 10) est générée avec un dispositif laser (11), et un faisceau laser pulsé est généré avec une longueur d'impulsion comprise entre 100 fs et 20 ps, une longueur d'onde comprise entre 200 nm et 1100 nm, un taux de répétition d'impulsions compris entre 1 kHz et 10 Mhz, un diamètre de foyer inférieur à 5 um et une densité de puissance supérieure à 10⁸ W/cm² et est incident au matériau de la lentille oculaire (1).
